# EUROPEAN PATENT SPECIFICATION

(11) **EP 0 670 826 B1**
(45) Date of publication and mention of the grant of the patent: **26.04.2000**
(21) Application number: 94903364.1
(22) Date of filing: 30.11.1993
(51) Int. Cl.: C07C 233/00, A61K 31/16

(54) **FATTY ACID DERIVATIVES AND PHARMACEUTICAL COMPOSITIONS CONTAINING THEM**
FETTSÄURE-DERIVATE UND DIESE ENTHALTENDE PHARMAZEUTISCHE PRÄPARATIONEN
DERIVES D'ACIDES GRAS ET COMPOSITIONS PHARMACEUTIQUES LES CONTENANT

(30) Priority: 30.11.1992 IL 10393292
(43) Date of publication of application: 13.09.1995
(73) Proprietor: YISSUM RESEARCH DEVELOPMENT COMPANY OF THE HEBREW UNIVERSITY OF JERUSALEM, Jerusalem 91042 (IL)
(72) Inventor: DEVANE, William, A. 5480 Wisconsin Avenue, Chevy Chase, MD 20815 (US); MECHOULAM, Raphael, 92 581 Jerusalem (IL); BEUER, Aviva, 93 707 Jerusalem (IL); HANUS, Lumir, 96 784 Jerusalem (IL)
(74) Representative: Weisert, Annekäte, Dipl.-Ing. Dr.-Ing.
(86) International application number: US9311625
(87) International publication number: WO9412466

(56) References cited:
- BE-A- 643 451
- FR-A- 1 476 596
- US-A- 4 619 938
- US-A- 4 877 789
- J. MED. CHEM. (1992), 35(19), 3584-6 CODEN: JMCMAR;ISSN: 0022-2623, 1992, XP002006310 JAIN, MAHENDRA K. ET AL: "Fatty acid amides: scooting mode-based discovery of tight-binding competitive inhibitors of secreted phospholipases A2"
- J. CHEM. SOC. C (1971), (22), 3821-7 CODEN: JSOOAX, 1971, XP002006311 GLILY-TERRY, S. ET AL: "Metalation reactions. X. Allylic metalation in the presence of amide groups Long range interactions"
- JOURNAL OF THE AMERICAN CHEMICAL SOCIETY, vol. 74, 1952, WASHINGTON DC US, pages 3423-3425, XP002006312 M. JACOBSON: "Constituents of Heliopsis Species.II. Synthesis of Compunds Related to Scabrin"

## Description

The invention relates to certain polyunsaturated fatty acid amides, and derivatives of these. Part of these are present in the brain, and part are products of synthesis. The novel pure compounds have a variety of pharmacological properties. They inhibit the specific binding of a cannabinoid probe to synaptosomal membranes. The compounds of the invention can be provided in radioactivity tagged form.

### BACKGROUND OF THE INVENTION

Arachidonic acid ethanolamide (anandamide) and similar compounds are constituents of the brain. Anandamide and certain of the compounds similar with same, bind to the cannabinoid receptor. The binding of the ananamide to the cannabinoid receptor is similar to the binding of Δ⁹-tetrahydrocannabinol. There exist in the body many mediators, which are derivatives of arachidonic acid, such as prostaglandins and leukotrienes, which are present as large families of related compounds. Certain of these do not bind to the cannabinoid receptor, and it was one of the aims of the present invention to provide and identify compounds which have pharmacological properties similar to the properties of anandamide.
The existence of a receptor and the high structural requirements for cannabinoid activity indicate the possible presence of a specific endogenous cannabinoid ligand.

U.S. patent 4,619,938 discloses alkanolamine derivatives and platelet aggregation inhibitors containing the same as an active ingredient. These derivatives possess platelet aggregation inhibitory activities and are effective in preventing diseases such as thrombosis. As typical compounds, U.S. patent 4,619,938 mentions N-5,8,11,14,17-eicosapentaenoyl-2-aminoethanol, N-nicotinoyl-2-aminoethyl-5,8,11,14,17-eicosapentaenoate or N-ethyl-N-5,8,11,14,17-eicosapentaenoyl-2-aminoethanol.

U.S. patent 4,877,789 discloses compounds of the formula

C₈H₁₇-(C≡C-CH₂)₃-CH₂CH₂COR

wherein R may be an amino group having the general formula

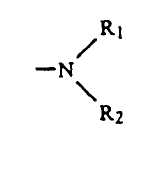

U.S. patent 4,877,789 further discloses the application of these compounds, on the one hand as a therapeutic agent in the treatment or prophylaxis of allergic diseases and in the treatment of dermatoses and inflammatory diseases, and on the other hand in cosmetic compositions.

U.S. patent 4,497,827 discloses tetrazole, acylhydroxylamine, hydroxymethylketone and amide derivatives of unsaturated fatty acids which are selective inhibitors of the enzymes lipoxygenase and cyclooxygenase involved in the production of pain, inflammation, bronchoconstriction and allergic reactions.

### SUMMARY OF THE INVENTION

Endogenous ligands for the cannabinoid receptor have not yet been identified. Arachidonylethanolamide, a new arachidonic acid derivative - named anandamide, was isolated from porcine brain. Its structure was determined by mass spectrometry and nuclear magnetic resonance spectroscopy and was confirmed by synthesis. It inhibits the specific binding of a labelled cannabinoid probe to synaptosomal membranes in a manner typical of competitive ligands, and produces a concentration-dependent inhibition of the electrically-evoked twitch response of the mouse vas deferens, a characteristic effect of psychotropic cannabinoids. Similar compounds were synthesized and their pharmacological properties were investigated.

Δ⁹-Tetrahydrocannabinol (Δ⁹-THC), the psychoactive constituent of Cannabis binds to a specific G-protein coupled receptor in the brain. Although the cannabinoid receptor in the rat and in the human has been cloned, its physiological function is unknown. The well established behavioral effects of THC and the abundance and anatomical localization of the receptor in the brain suggest a role for the receptor in the control of movement, memory, emotions and pain modulation, amongst other activities.

### DESCRIPTION OF PREFERRED EMBODIMENTS

The existence of a receptor and the high structural requirements for cannabinoid activity indicate the possible presence of a specific endogenous cannabinoid ligand.
To screen for endogenous cannabinoid compounds there were tested brain-derived fractions using a centrifugation radioligand binding assay. Tritiated HU-243 (11-hydroxyhexahydrocannabinol-3-dimethylheptyl homolog) with a K_{D} of 45 pM in rat synaptosomal membranes, was used as the probe. Organic soluble extracts of porcin brain were first chromatographed according to standard protocols for the separation of lipids. Pig brains were homogenized in chloroform and/or methanol and centrifuged at 13,000 x g. The organic soluble extract was fractionated over silica (Kieselgel 60, 70-230 mesh), following elution schemes used to separate the major classes of lipids [C.C.Sweeley, Methods Enzymol. 14, 254 (1969); J.C. Dittmer and M.A. Wells, ibid. p. 482]. Many of the initial fractions isolated from brain inhibited the binding of [³H]HU-243 to the cannabinoid receptor. Particular attention was paid to the binding of [³H]HU-243 to the siliconized polypropylene microfuge tubes in which the assay was conducted. Normally, about 15-20% of the added [³H]HU-243 adheres to the microfuge tube, with the amount increasing slightly when unlabelled cannabinoid drugs displace the radioligand from the receptor. When monitoring the three-way equilibrium of [³H]HU-243 among the synaptosomal receptors, the solution and the microfuge tube, we observed that all the crude brain fractions which inhibited the binding of [³H]HU-243 to the receptors also inhibited the binding of the radioligand to the microfuge tube. Several promising fractions were purified using both low and medium pressure column chromatography as well as thin layer chromatography (tlc). A combination of normal phase and reverse phase systems was employed. Tlc: Rf 0.65 on an analyticial RP-18 plate (Merck), eluted with methanol-dichloromethane, 4:1: developed twice-first solvent front: 3.1 cm, second solvent front: 7.4 cm. [W.A. Devane, L. Hanus, Mechoulam, Proceed. 5th Nordic Neuroscience Meet., Publ. Univ. Kuopio Med. p. 198 (1991)]. Anandamide elutes from a silica column (Kieselgel 60, 40-63 um, Merck) with methanol: chloroform (2:98). It elutes from a reverse-phase column (RP-C , 40-63 µm, Sigma) with methanol:
water (88:12).

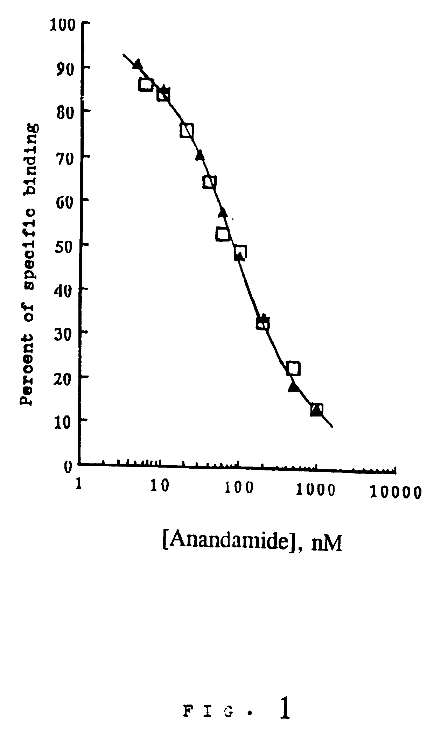

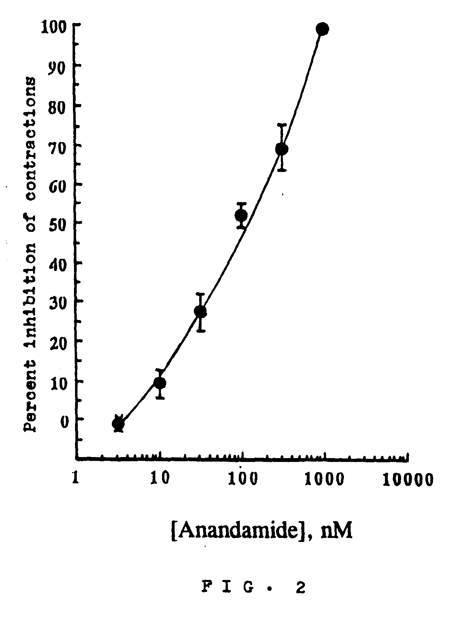

A compound was recovered (0.6 mg from 4.5 kg of brain), named anandamide, which shows one spot on tlc and elutes mainly as one peak on gas chromatography (GC) using a mass spectrometer as a detector. Anandamide inhibits the specific binding of [³H]HU-243 to synaptosomal membranes in a manner typical of competitive ligands with a K_{D} of 52 + 1.8 nM (n=3) (Fig. 1). In this system the K_{D} of Δ⁹-THC was 46 + 3 nM.
Results from previous experiments, in which we compared the inhibitory effects of the 1,1-dimethyiheptyl homologs of (+)- and (-)-11-hydroxy-delta-8-tetrahydrocannabinol on the electrically-evoked twitch response of the mouse vas deferens, indicate that this preparation is suitable as a model for investigating the mode(s) of action of psychotropic cannabinoids. R.G. Pertwee, L.A. Stevenson, D.B. Elrick, R. Mechoulam, A.D. Corbett, Brit. J. Pharmacol. 105, 980 (1992). Anandamide produced a concentration-dependent inhibition of the twitch response (Fig. 2). The inhibition was not reversed by naloxone (300 nM). The levels of inhibition are comparable to those of binding to the receptor.
The structure of anandamide was established by mass spectrometry (MS) and nuclear magnetic resonance (NMR) spectroscopy. Additional data were obtained from the GC-MS and CID measurements of the trimethylsilyl (TMS) derivative of the material. The results suggest that anandamide is an ethanolamide of a tetraenic C₂₀ fatty acid.

Support for the above structure was found in the behavior of anandamide under GC-MS conditions.

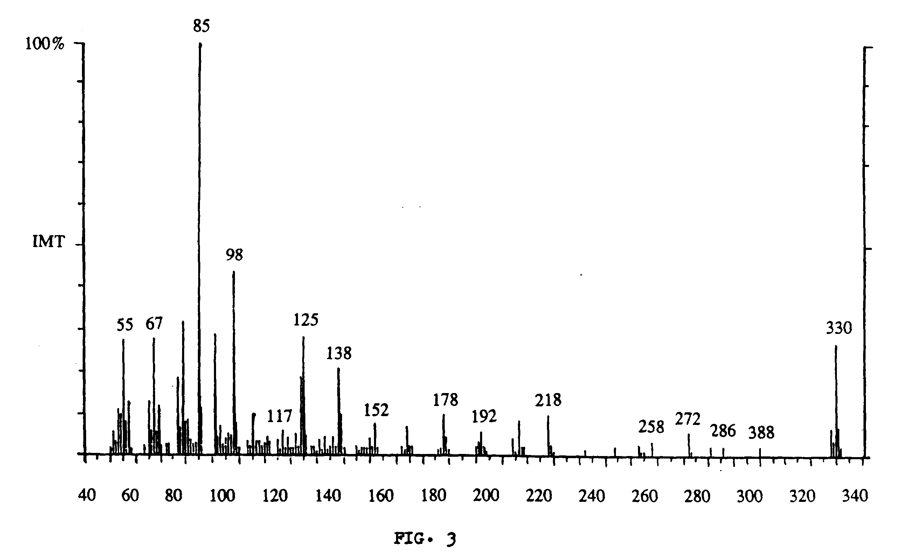

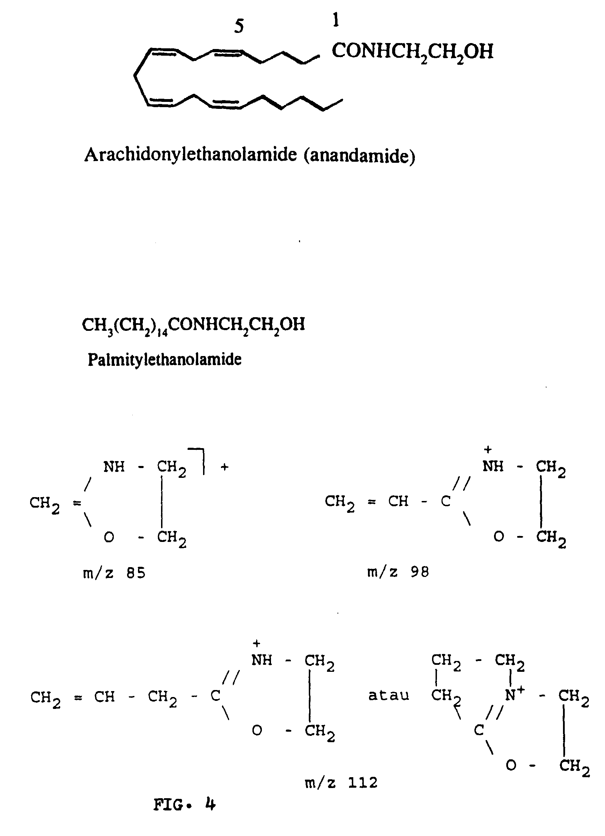

Thermal dehydration gives rise to m/z 329 M+ ion upon electron ionization (EI) and to m/z 330 MH+ under C1.
Both self-C1 m/z 330 MH+ and m/z 329 M+ are formed under EI conditions in an ion trap instrument (Fig. 3). The fragmentation pattern of the dehydration products was similar in the low mass range of the EI mass spectra of both anandamide and palmitylethanolamide: m/z 85 (McLafferty rearrangement ion) and m/z 98 (product of a γ-cleavage) (Fig. 4). The EI mass spectrum of dehydrated palmitylethanolamide exhibits an m/z 112 ion corresponding to a δ-cleavage fragment. The absence of this ion in the EI mass spectrum obtained in the GC-MS analysis of anandamide suggests the presence of the first double bond in the tetraenic acid at position 5 (as in arachidonylethanolamide which would not be expected to yield a - cleavage product) (Fig. 4).

¹H NMR spectra were recorded. The peaks attributed to double bond protons (δ, 5.30-5.45, mult) were coupled with those of protons which have the chemical shifts of doubly allylic ones (δ, 2.75-2.90, mult). Such doubly allylic protons are typical for numerous naturally occuring all-cis, nonconjugated, polyunsaturated fatty acids such as linoleic and arachidonic acids. Three pairs of protons were observed between δ2.01-2.27, which we attributed to 2 allylic methylene groups and 1 methylene group α to a carbonyl moiety. Only one methyl group was observed (0.88,t). The peaks observed for 2 protons at 3.42 (N-CH t), 2 protons at 3.72 (0-CH₂,t) and 2 protons at 2.20 (COCH₂, t) are similar in chemical shifts and spin coupling patterns to peaks observed in the NMR spectrum of synthetic palmitylethanolamide. The peaks for N-CH₂ and O-CH₂ were coupled.

A juxtaposition of the above analytical data led us to conclude that the structure of anandamide is that of arachidonyl ethanolamide [5,8,11,14-icosatetraenamide, (N,-2-hydroxyethyl)-(all-Z)] a novel chemical entity.
This conclusion was confirmed by synthesis. Arachidonyl chloride, prepared from arachidonic acid and oxalyl chloride (21), in methylene chloride, was added at 0 degrees C, under a nitrogen athmosphere to ethanolamine (in a ten fold molar excess) in methylene chloride. After 15 min the reaction was washed with water, dried, and the product (ca 90% yield) was purified by silica column chromatography (eluted with 2% methanol in chloroform) to give rachidony- lethanolamide, an oil, in 97% purity (by GC-MS). Synthetic arachidonylethanolamide was identical with the product obtained on tlc (10), NMR (300 MHz) and GC-MS (retension time and fragmentation pattern) (Fig. 3). Synthetic anandamide binds to the cannabinoid receptor K_{I} = 39 ± 5.0 nM (n=3).

The novel purified compound anandamide seems to be present as brain constituent. It is possible that this compound is present as a complex with another compound or in any other form, but according to the present invention it has been established that the compound defined herein as ananamide is characterized by the properties set out herein. Similar compounds, defined herein, are characterized by essentially equivalent properties and are part of the present invention.

This invention also relates to tagged form of such compounds, which can be used in a variety of research projects and in assays.

The invention further relates to pharmaceutical compositions containing an effective quantity of one of the compounds defined herein.

Following the synthetic method described for arachidonylethanolamide, the ethanolamides of the following unsaturated fatty acids were prepared.

| Systematic Name | Trivial Name | Shorthand designation |
|---|---|---|
| 9,12-octadecadienoic* | linoleic | 18:2 (n-6) |
| 4,7,10,13,16,19-docosahexaenoic | - | 22:6 (n-3) |

| | | |
|---|---|---|
| * The double-bond configuration in each instance is cis. | | |

These ethanolamide derivatives have antiinflammatory analgetic, antiglaucoma and antiemetic activity (see Biological Results). In order to obtain 14C labeled compounds of importance in biological studies the above described synthetic procedure was repeated with 14C-labeled arachidonic acid to give 14C-arachidonyl ethanolamide, 50 mC1/mmol. With ³ H-arachidonic acid we obtained ³H-arachidonylethanol amide, 150 Ci/mmol. When we used 14C-labeled ethanolamide we obtained 14C-arachidonylethanolamide, 50 mCi/mmol.

### Biological Results

Analgesia. The novel compounds were tested in the standard hot plate test.

Represenatative results are presented in Table I. The compounds were dissolved in a detergent (Emulphor): ethanol: saline (5:5:90) and administered by intravenous injection in the tail vein with an injection volume of 0.1 ml/10g of body weight.

### Antiemetic activity

The compounds also tested in pigeons against emesia caused by an antieoplastic drug (cisplatin) according to the method described in Feigenbaum et al., Eur. J. Pharmacol. 169, 159-165 (1989). Representative results are presented in Table I.
The compounds were dissolved as described above for the tests on analgesia, and administered subcutaneously. The injection volume was 1.0 ml/kg body weight.

### Antiglaucoma activity

The compounds were also tested for antiglaucoma activity in rabbits with stable glaucoma induced by δ-chymotrypsin injection into the eye as described in detail in R. Mechoulam et al., On the therapeutic possiblities of some cannabinoids in "The Therapeutic Potential of Marihuana" (eds S. Cohen. R.C. Stillman) Plenum Press, New York, 1975. pp. 35-48). The compounds were compared in activity to that of pilocarpine (a standard antiglaucoma drug) which at 0.01% aq. solution reduces the intraocular pressure (I.O.P.) and delays recovery to half time of the original I.O.P. about 30 hours.
The fatty acid ethanol amides administered to the eye in 0.1% solution were less active than pilocarpine and delayed recovery to half time of the original I.O.P between 2 and 10 hours.
The compounds were dissolved as described for analgesia and further diluted with saline to obtain the required concentration.

### Antiinflammatory activity

The compounds were tested by a method reported by W. Calhoun et al., Agents and Actions, 21, 306-a309 (1987).
Water was substituted for mercury as the displacement medium. PAF (1.0 µg) or arachidonic acid (1.0mg) dissolved in 50 µl of 5% ethanol in saline was injected s.c. into the plantar surface of the right hind paw of female mice (20-25g). The mice were under ether anesthesia during this procedure. The volume of the right foot was measured to the level of the lateral malleous by water displacement before treatment and 15 min after PAF injection or 30 min after arachidonic injection.
The change in paw volume was calculated for each mouse.
The results for two of the tested ethanolamides are presented in Table II.

### Dosage

The effective doses for humans are between 1-100 mg total daily dose, by injection or by oral administration.

**Table I**

| Analgesia and Vomiting Reduction | | |
|---|---|---|
| Compound | Analgesia ED=₅₀ (mg/kg)- | Reduction of vomiting(50%)= (mg/kg) |
| Arachidonylethanolamide | 4.2 | 2.5 |
| 4,7,10,13,16,19-docosahexaenylethanolamide | 6.1 | 4.8 |

| | | |
|---|---|---|
| - in mice. For details of administration see Text | | |
| = in pigeons. For details ofadminstration see Text. | | |

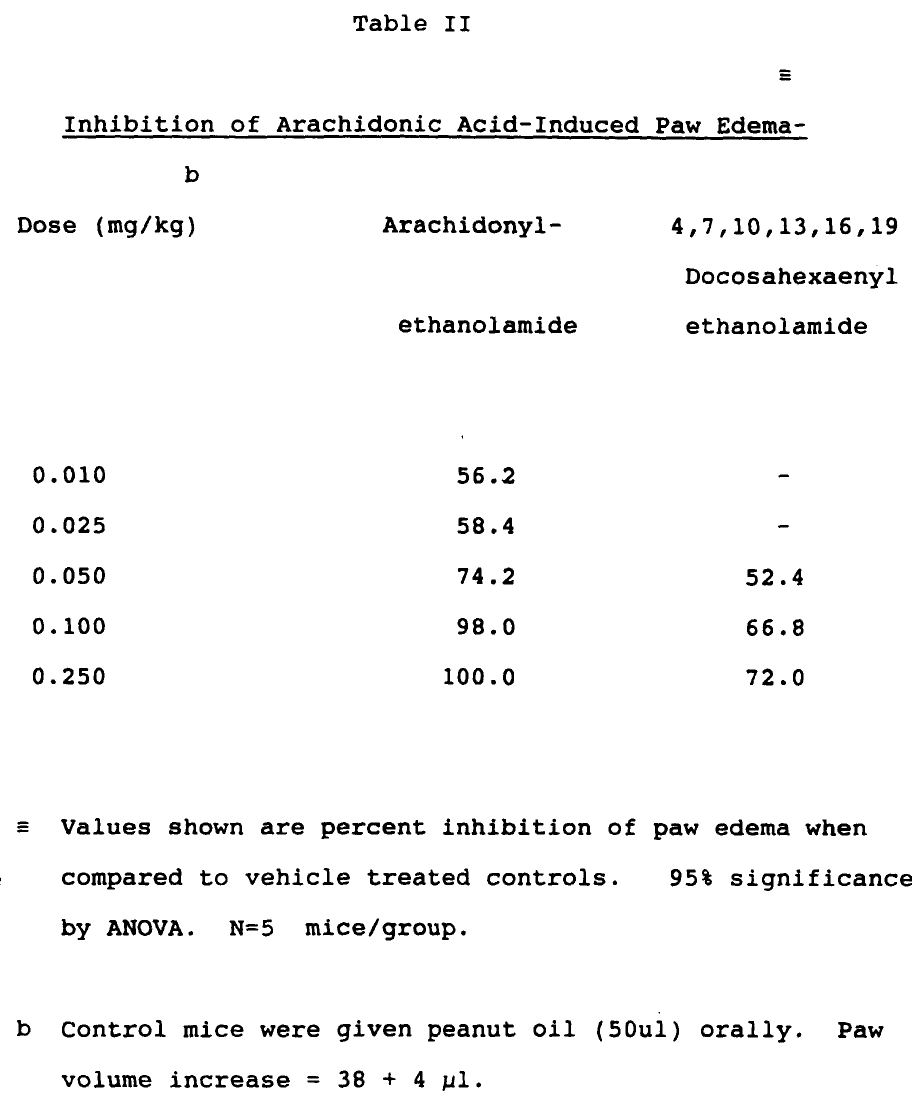

### BRIEF DESCRIPTION OF THE FIGURES

The invention is illustrated with reference to the enclosed Figures, wherein:
Fig. 1 illustrates the competitive inhibition of [³H] HU-243 binding by natural anandamide;
Fig. 2 illustrates inhibition of vas deferens twitch response by natural anandamide;
Fig. 3 is a GC-MS spectrum of anandamide;
Fig. 4 illustrates the structure of some compounds described;

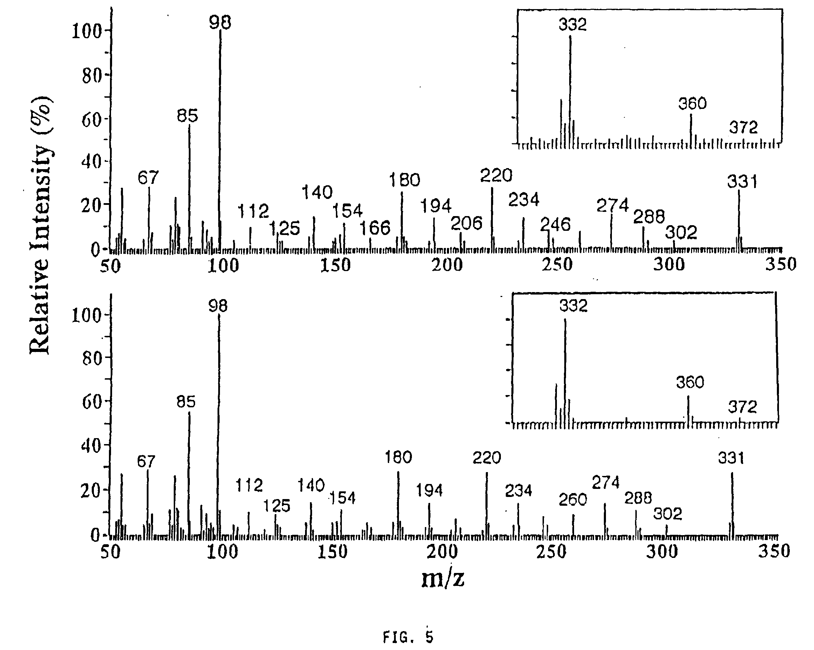

Fig. 5 EI GC-MS spectra of dehydrated homo-γ-linolenylethanolamine (5);

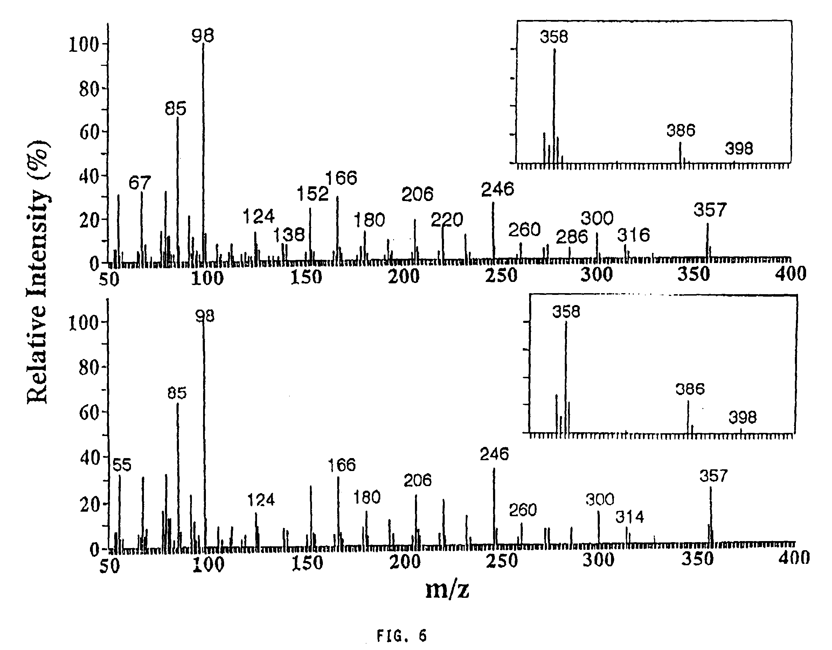

Fig. 6 EI GC-MS spectra of dehydrated docosatetraenylethanolamine, Compound (6);

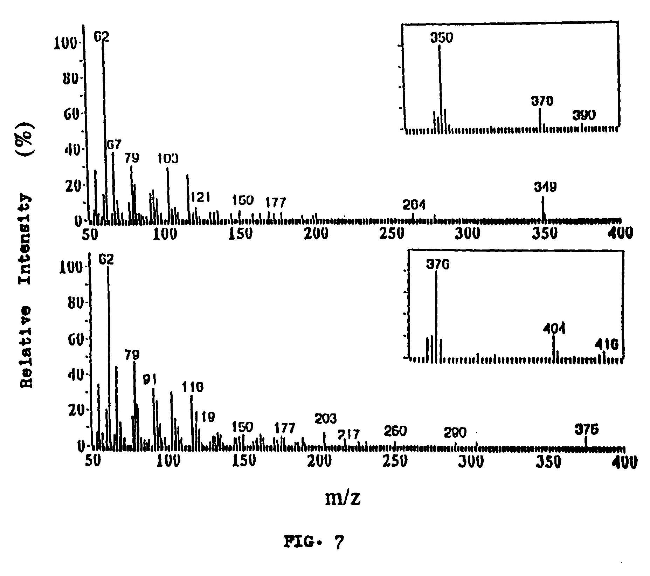

Fig. 7 EI GC-MS spectra of homo-δ-linolenylethanolamine (5) and of docosatetraenylethanolamine, (top and bottom);

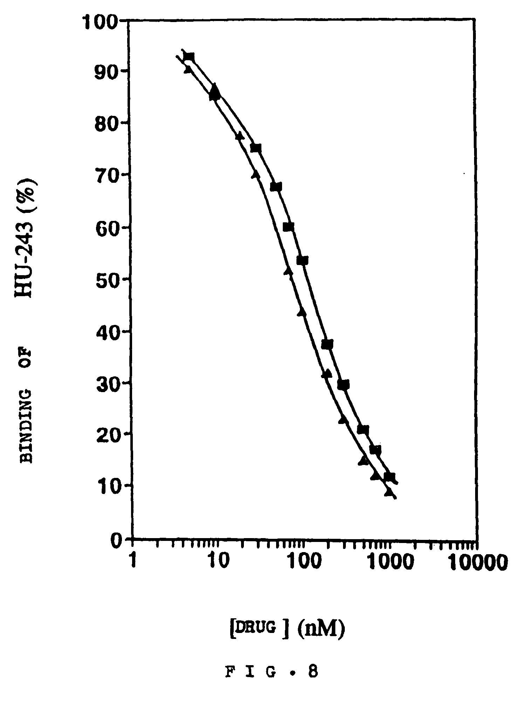

Fig. 8 illustrates competitive binding of [³H]HY-243 by compound (5), squares, and by compound (6), triangles;

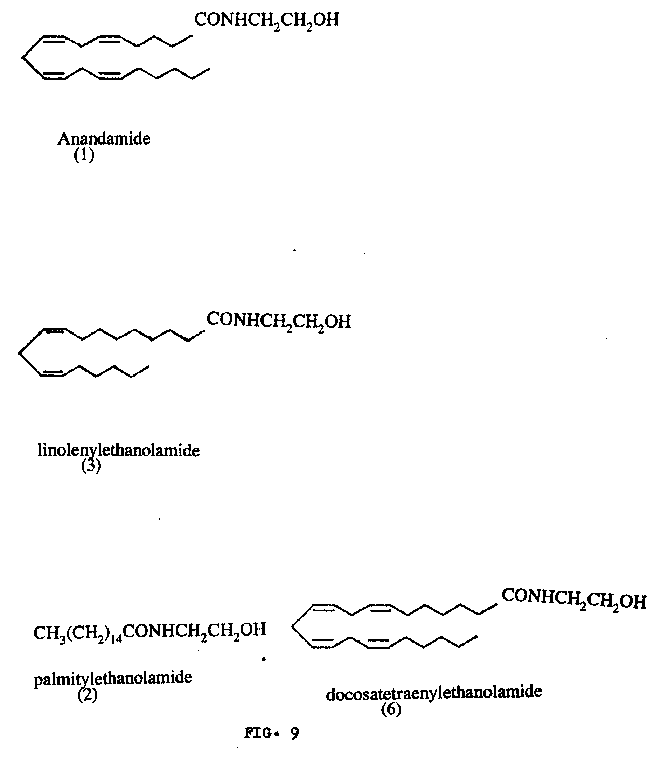

Fig. 9 is a Formula sheet of some compounds of the invention.

### Legends

Fig. 1. Competitive inhibition of [ H]HU-243 binding by natural anandamide. Synaptosomal membranes were prepared from rat whole brain-minus brainstem (Sprague-Dawley males, 430-470g). [³H]HU-243 (45-55 pM) was incubated with synaptosomal membranes (protein content 3-4 µg) for 90 min at 30°C with either the indicated concentrations of anandamide or the vehicle alone (fatty acid-free bovine serum albumin, final concentration-0.5 mg/ml). Bound and free radioligand were separated by centrifugation (see ref. 8 for full details of the assay). The data were normalized to 100% of specific binding, which was determined with 50 nM unlabeled HU-243. The amount of specific binding was 77-82% of the total radioactivity bound to the membranes. Data points (□, ▲) represent the average of triplicate determinations from two independent experiments. K_{I} value (mean ± SE, n=3) was determined using the Ligand program; K_{I} = 52 ± 1.8 nM.

Fig. 2. Inhibition of vas deferens twitch response by natural anandamide. Vasa deferentia obtained from MFI mice were mounted in 4 ml siliconized organ baths under an initial tension of 0.5 g. The baths contained Mg++ -Free Krebs solution which was kept at 37 C and bubbled with 95% 0₂ and 5% CO₂ . Tissues were stimulated supramaximally with 0.5 s trains of 3 pulses (train frequency 0.1 Hz: pulse duration 0.5 ms). Isometric contractions were recorded. Natural anandamide was dispersed in Tween 80 and saline (see ref. 12) and added in volumes of 40 µl. Tween 80 did not inhibit the twitch response at the maximum bath concentration used (0.63 µg/ml; n=6).

Fig. 3. GC-MS spectrum of anandamide on an ion trap instrument. Anandamide undergoes thermal dehydration under these conditions (see text) hence the above spectrum in actuality is that of the M⁺ ion of the corresponding 2-oxazoline.

Fig. 4. Structures of anandamide and of palmitylethanolamide and of the dihydro- and tetrahydrooxazole ion fragments formed from these fatty acid ethanolamides on thermal dehydration under GC-MS conditions (see text). The m/z 112 ion is formed only from palmitylethanolamide.

| **COMPOUNDS ISOLATED FROM THE BRAIN AND SYNTHESIZED**. | |
|---|---|
| NAME OF COMPOUND | Ki |
| cis-7,10,13,16-docosatetraenoylethanolamide | 34.4 ± 3.2 nM |
| anandamide | 39.0 ± 5.0 nM |

| **SYNTHETIC COMPOUNDS, PREPARED AND TESTED.** | |
|---|---|
| NAME OF COMPOUND | Ki |
| arachidonoyl-β-dimethylethanolamide | 161.8 ± 34.1 nM |

| Compound 6: | |
|---|---|
| Calcd for C₂₄H₄₁NO₂ (m.w. 375): | C,76.75 |
| | H,11.00 |
| | N, 3.73 |
| Found | C,76.92 |
| | H,11.34 |
| | N, 3.60 |

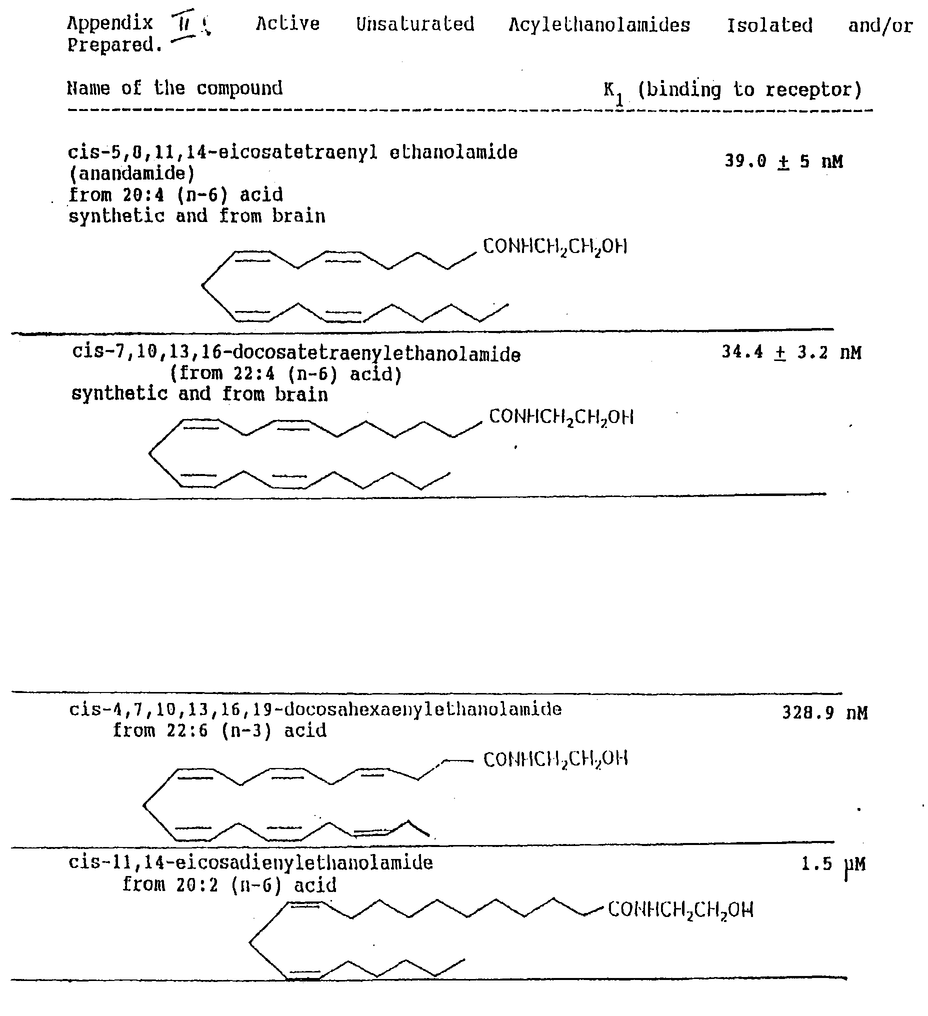

### REFERENCES

1. Devane, W.A. et al., Hanus, L.; Breuer, A.; Pertwee, R.G.; Stevenson, L.A.; Griffin, G.; Gibson, D.; Mandelbaum, A.; Etinger, A.; Mechoulam, R. Isolation and Structure of a Brain Constituent that Binds to the Cannabinoid Receptor. Science, 1992, 258, 1946-1949;
2. Fride, E.; Mechoulam, R. Pharmacological Activity of the Cannabinoid Agonist Anandamide, a Brain Constituent. Eur. J. Pharmacol. 1993, 231, 313-314;
3. Vogel, Z.; Barg, J.; Levy, R.; Saya, D.; Heldman, E.; Mechoulam, R. Anandamide, a Brain Endogenous Compound, Interacts Specifically with Cannabinoid Receptors and Inhibits Adenylate Cyclase. J. Neurochem. (in press);
4. Bachur, N.R.; Masek, K.; Melmon, K.L.; Udenfriend, S. Fatty Acid Amides of Ethanolamine in Mammalian Tissues. J. Biol. Chem. 1965, 240, 1019-1024;
5. Agranoff, B.W. In Basic Neurochemistry; Seigel, G., Agranoff, B., Albers, R.W., Molinoff, P., Eds; Raven Press: New York, 1989;
6. Devane, W.A. et al., Breuer, A.; Jarbe, T.U.C.; Eisen, M.; Mechoulam, R. A Novel Probe for the Cannabinoid Receptor. J. Med. Chem. 1992, 35, 2065-2069.

## Claims

1. Compounds of the general formula 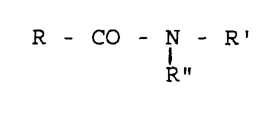 wherein R is the alkenyl moiety of a polyunsaturated fatty acid of 16 to 28 carbon atoms, with 2 to 6 double bonds, with the first double bond at the C-3, C-6 or C-9 position, counting from the non-carboxyl part of the molecule, where R'' is hydrogen and R' is -(CH₂)ₘOH, where m is 2 and acid addition salts and complexes of these with the proviso that the polyunsaturated fatty acid is not a trienic or pentaenic higher fatty acid.

2. A compound according to claim 1 wherein all double bonds of the polyunsaturated fatty acid are in the cis-configuration.

3. A compound according to claim 1 or 2, where the alkenyl moiety is an octadecadienoic, docosahexaenoic or eicosatetraenoic moiety.

4. A compound according to claim 1 or 2, selected from arachidonylethanolamide, cis-7,10,13,16-docosatetraenoylethanolamide.

5. An antiinflammatory, anti-asthmatic, analgetic, anti-emetic, antiglaucoma, anti-migraine, anti-spasticity, mood stimulating and symptoms of multiple sclerosis ameliorating pharmaceutical composition, which contains as active ingredient an effective quantity of a compound defined in any of claims 1 to 4.

6. A composition according to claim 5, where the active ingredient is a compound defined in claim 4.

7. A composition according to claim 5 or 6, where the unit dosage for human administration is from about 1 mg to about 100 mg of the active compound.

8. A compound defined in any of claims 1 to 4, in radioactive tagged form.

9. A compound according to claim 8, where the tag is ³H or ¹⁴C.

10. A composition for binding to the cannabinoid receptor in the brain, comprising an effective quantity of a compound defined in any of claims 1 to 4.

11. Use of compounds of the general formula 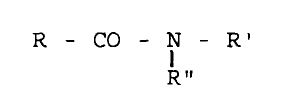 wherein R is the alkenyl moiety of a polyunsaturated fatty acid of 16 to 28 carbon atoms, with 2 to 6 double bonds, with the first double bond at the C-3, C-6 or C-9 position, counting from the non-carboxyl part of the molecule, where R'' is hydrogen and R' is -(CH₂)ₘOH, where m is 2 and acid addition salts and complexes of these for the preparation of a medicament having antiinflammatory, anti-asthmatic, analgetic, anti-emetic, antiglaucoma, anti-migraine, anti-spasticity, mood stimulating and symptoms of multiple sclerosis ameliorating activity.

12. Use according to claim 11, wherein all the double bonds of the polyunsaturated fatty acids of the compounds as defined in claim 11 are in the cis configuration.

13. Use according to any of claims 11 to 12 wherein the alkenyl moiety of the compounds defined in these claims is an octadecadienoic, actadecatrienoic, eicosapentaenoic, docosahexaenoic, eicosatrienoic or eicosatetraenoic moiety.

14. Use according to any of claims 11 to 13 wherein the compounds as defined in these claims are selected from arachidonylethanolamide, cis-7,10,13,16-docosatetraenoylethanolamide, homo-δ-linolenoylethanolamide.

15. Use according to any of claims 11 to 14, wherein the unit dosage for human administration is from about 1 mg to about 100 mg of the compound.

16. Use of compounds of the general formula 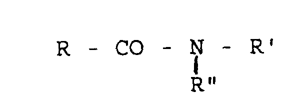 wherein R is the alkenyl moiety of a polyunsaturated fatty acid of 16 to 28 carbon atoms, with 2 to 6 double bonds, with the first double bond at the C-3, C-6 or C-9 position, counting from the non-carboxyl part of the molecule, where R'' is hydrogen and R' is -(CH₂)ₘOH, where m is 2 and acid addition salts and complexes of these for the preparation of a composition for binding to the cannabinoid receptor in the brain.

17. Use according to claim 16, wherein all the double bonds of the polyunsaturated fatty acids of the compounds as defined in claim 11 are in the cis configuration.

18. Use according to any of claims 16 to 17 wherein the alkenyl moiety of the compounds defined in these claims is an octadecadienoic, actadecatrienoic, eicosapentaenoic, docosahexaenoic, eicosatrienoic or eicosatetraenoic moiety.

19. Use according to any of claims 16 to 18 wherein the compounds as defined in these claims are selected from arachidonylethanolamide, cis-7,10,13,16-docosatetraenoylethanolamide, homo-δ-linolenoylethanolamide.

## Patentansprüche

1. Verbindungen der allgemeinen Formel 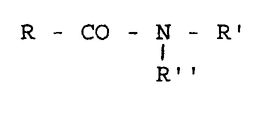 worin R eine Alkenylgruppierung einer mehrfach ungesättigten Fettsäure mit 16 bis 28 Kohlenstoffatomen und mit 2 bis 6 Doppelbindungen ist, wobei sich die erste Doppelbindung an der C-3-, C-6- oder C-9-Position, gezählt ausgehend von dem Nicht-Carboxylanteil des Moleküls, befindet und wobei R'' ein Wasserstoffatom ist und R' -(CH₂)ₘOH ist, wobei m für 2 steht, sowie Säureadditionssalze und Komplexe davon, mit der Maßgabe, daß die mehrfach ungesättigte Fettsäure nicht eine trienische oder pentaenische höhere Fettsäure ist.

2. Verbindung nach Anspruch 1, dadurch **gekennzeichnet,** daß alle Doppelbindungen der mehrfach ungesättigten Fettsäure sich in der cis-Konfiguration befinden.

3. Verbindung nach Anspruch 1 oder 2, dadurch **gekennzeichnet,** daß der Alkenylrest ein Octadecadien-, Docosahexaen- oder Eicosatetraenrest ist.

4. Verbindung nach Anspruch 1 oder 2, ausgewählt aus Arachidonylethanolamid, Cis-7,10,13,16-docosatetraenoylethanolamid.

5. Antiinflammatorisches, antiasthmatisches, analgetisches, antiemetisches, antiglaucomatöses, gegen Migräne gerichtetes, antispastisches, stimmungsstimulierendes und Symptome der Multiplen Sklerose verbesserndes pharmazeutisches Präparat, welches als Wirkstoff eine wirksame Menge einer Verbindung nach einem der Ansprüche 1 bis 4 enthält.

6. Präparat nach Anspruch 5, dadurch **gekennzeichnet,** daß der Wirkstoff eine Verbindung nach Anspruch 4 ist.

7. Präparat nach Anspruch 5 oder 6, dadurch **gekennzeichnet,** daß die Einheitsdosierung für die Verabreichung an den Menschen von etwa 1 mg bis etwa 100 mg des Wirkstoffes reicht.

8. Verbindung nach einem der Ansprüche 1 bis 4 in radioaktiv markierter Form.

9. Verbindung nach Anspruch 8, dadurch **gekennzeichnet,** daß die Markierung ³H oder ¹⁴C ist.

10. Mittel für die Bindung an den Cannabinoid-Rezeptor im Gehirn, umfassend eine wirksame Menge einer Verbindung nach einem der Ansprüche 1 bis 4.

11. Verwendung von Verbindungen der allgemeinen Formel 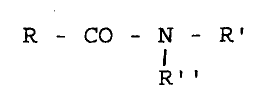 worin R ein Alkenylrest einer mehrfach ungesättigten Fettsäure mit 16 bis 28 Kohlenstoffatomen und mit 2 bis 6 Doppelbindungen ist, wobei sich die erste Doppelbindung an der C-3-, C-6- oder C-9-Position, gezählt ausgehend von dem Nicht-Carboxylanteil des Moleküls, befindet und wobei R'' ein Wasserstoffatom ist und R' -(CH₂)ₘOH ist, wobei m für 2 steht, sowie Säureadditionssalze und Komplexe davon zur Herstellung eines Medikaments, welches antiinflammatorische, antiasthmatische, analgetische, antiglaucomöse, gegen Migräne gerichtete, antispastische, stimmungsstimulierende und Symptome der Multiplen Sklerose verbessernde Aktivitäten besitzt.

12. Verwendung nach Anspruch 11, dadurch **gekennzeichnet,** daß alle Doppelbindungen der mehrfach ungesättigten Fettsäuren der Verbindungen nach Anspruch 11 sich in Cis-Konfiguration befinden.

13. Verwendung nach einem der Ansprüche 11 bis 12, dadurch **gekennzeichnet,** daß der Alkenylrest der Verbindungen nach diesen Ansprüchen ein Octadecadien-, Actadecatrien-, Eicosapentaen-, Docosahexaen-, Eicosatrien- oder Eicosatetraenrest ist.

14. Verwendung nach einem der Ansprüche 11 bis 13, dadurch **gekennzeichnet,** daß die in diesen Ansprüchen definierten Verbindungen ausgewählt sind aus Arachidonylethanolamid, Cis-7,10,13,16-docosatetraenoylethanolamid, Homo-δ-linolenoylethanolamid.

15. Verwendung nach einem der Ansprüche 11 bis 14, dadurch **gekennzeichnet,** daß die Einheitsdosierung für die Verabreichung an den Menschen von etwa 1 mg bis etwa 100 mg der Verbindung reicht.

16. Verwendung von Verbindungen der allgemeinen Formel 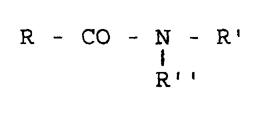 wobei R ein Alkenylrest einer mehrfach ungesättigten Fettsäure mit 16 bis 28 Kohlenstoffatomen und mit 2 bis 6 Doppelbindungen ist, wobei sich die erste Doppelbindung an der C-3-, C-6- oder C-9-Position, gezählt ausgehend von dem Nicht-Carboxylanteil des Moleküls, befindet und wobei R'' ein Wasserstoffatom ist und R' -(CH₂)ₘOH ist, wobei m für, 2 steht, sowie Säureadditionssalze und Komplexe davon zur Herstellung eines Mittels für das Binden an den Cannabinoid-Rezeptor im Gehirn.

17. Verwendung nach Anspruch 16, dadurch **gekennzeichnet,** daß alle Doppelbindungen der mehrfach ungesättigten Fettsäuren der Verbindungen nach Anspruch 11 sich in der Cis-Konfiguration befinden.

18. Verwendung nach einem der Ansprüche 16 bis 17, dadurch **gekennzeichnet,** daß der Alkenylrest der Verbindungen nach diesen Ansprüchen ein Octodecadien-, Actadecatrien-, Eicosapentaen-, Docosahexaen-, Eicosatrien- oder Eicosatetraenrest ist.

19. Verwendung nach einem der Ansprüche 16 bis 18, dadurch **gekennzeichnet,** daß die Verbindungen nach diesen Ansprüchen ausgewählt sind aus Arachidonylethanolamid, Cis-7,10,13,16-docosatetraenoylethanolamid, Homo-δ-linolenoylethanolamid.

## Revendications

1. Composés de la formule générale : 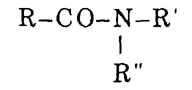 dans laquelle R est la partie alcényle d'un acide gras polyinsaturé de 16 à 28 atomes de carbone, avec 2 à 6 doubles liaisons, dont la première double liaison est en position C-3, C-6 ou C-9, en comptant à partir de la partie non carboxylique de la molécule, dans laquelle R'' est un atome d'hydrogène et R' est un groupe -(CH₂)ₘOH, dans lequel m est 2, et sels d'addition d'acide et complexes de ceux-ci, à condition que l'acide gras polyinsaturé ne soit pas un acide gras supérieur triénique ou pentaénique.

2. Composé suivant la revendication 1, dans lequel toutes les doubles liaisons de l'acide gras polyinsaturé sont en configuration cis.

3. Composé suivant les revendications 1 ou 2, dans lequel la partie alcényle est une partie octadécadiénoïque, docosahexaénoïque ou eicosatétraénoïque.

4. Composé suivant les revendications 1 ou 2, choisi parmi l'arachidonyléthanolamide, le cis-7,10,13,16-docosatétraénoyléthanolamide.

5. Composition pharmaceutique anti-inflammatoire, anti-asthmatique, analgésique, anti-émétique, antiglaucome, antimigraineuse, antispasmodique, stimulant l'humeur et améliorant les symptômes de la sclérose en plaques, qui contient en tant que composant actif une quantité efficace d'un composé défini dans l'une quelconque des revendications 1 à 4.

6. Composition suivant la revendication 5, dans laquelle le composant actif est un composé défini dans la revendication 4.

7. Composition suivant les revendications 5 ou 6, dans laquelle la dose unitaire pour l'administration à l'homme est comprise entre environ 1 mg et environ 100 mg du composé actif.

8. Composé suivant l'une quelconque des revendications 1 à 4, sous une forme à marqueur radioactif

9. Composé suivant la revendication 8, dans lequel le marqueur est ³H ou ¹⁴C.

10. Composition pour une liaison au récepteur de cannabinoïde dans le cerveau, comprenant une quantité efficace d'un composé défini dans l'une quelconque des revendications 1 à 4.

11. Utilisation de composés de la formule générale : 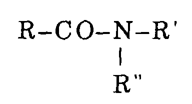 dans laquelle R est la partie alcényle d'un acide gras polyinsaturé de 16 à 28 atomes de carbone, avec 2 à 6 doubles liaisons, dont la première double liaison est en position C-3, C-6 ou C-9, en comptant à partir de la partie non carboxylique de la molécule, dans laquelle R'' est un atome d'hydrogène et R' est un groupe -(CH₂)ₘOH, dans lequel m est 2, et de sels d'addition d'acide et de complexes de ceux-ci, pour la préparation d'un médicament ayant des activités anti-inflammatoire, anti-asthmatique, analgésique, anti-émétique, antiglaucome, antimigraineuse, antispasmodique, stimulant l'humeur et améliorant les symptômes de la sclérose en plaques.

12. Utilisation suivant la revendication 11, dans laquelle toutes les doubles liaisons des acides gras polyinsaturés des composés suivant la revendication 11 sont en configuration cis.

13. Utilisation suivant l'une quelconque des revendications 11 à 12, dans laquelle la partie alcényle des composés définis dans ces revendications est une partie octadécadiénoïque, octadécatriénoïque, eicosapentaénoïque, docosahexaénoïque, eicosatriénoïque ou eicosatétraénoïque.

14. Utilisation suivant l'une quelconque des revendications 11 à 13, dans laquelle les composés définis dans ces revendications sont choisis parmi l'arachidonyléthanolamide, le cis-7,10,13,16-docosatétraénoyléthanolamide, l'homo-δ-linolénoyléthanolamide.

15. Utilisation suivant l'une quelconque des revendications 11 à 14, dans laquelle la dose unitaire pour l'administration à l'homme est comprise entre environ 1 mg et environ 100 mg du compose.

16. Utilisation de composés de la formule 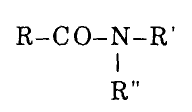 générale : dans laquelle R est la partie alcényle d'un acide gras polyinsaturé de 16 à 28 atomes de carbone, avec 2 à 6 doubles liaisons, dont la première double liaison est en position C-3, C-6 ou C-9, en comptant à partir de la partie non carboxylique de la molécule, dans laquelle R'' est un atome d'hydrogène et R' est un groupe -(CH₂)ₘOH, dans lequel m est 2, et de sels d'addition d'acide et de complexes de ceux-ci, pour la préparation d'une composition pour une liaison au récepteur de cannabinoïde dans le cerveau.

17. Utilisation suivant la revendication 16, dans laquelle toutes les doubles liaisons des acides gras polyinsaturés des composés définis suivant la revendication 11 sont en configuration cis.

18. Utilisation suivant l'une quelconque des revendications 16 à 17, dans laquelle la partie alcényle des composés définis dans ces revendications est une partie octadécadiénoïque, octadécatriénoïque, eicosapentaénoïque, docosahexaénoïque, eicosatriénoïque ou eicosatétraénoïque.

19. Utilisation suivant l'une quelconque des revendications 16 à 18, dans laquelle les composés définis dans ces revendications sont choisis parmi l'arachidonyléthanolamide, le cis-7,10,13,16-docosatétraénoyléthanolamide, l'homo-δ-linolénoyléthanolamide.
